(19) Europäisches Patentamt
European Patent Office
Office européen des brevets

(11) Veröffentlichungsnummer: **0 007 075**
**B1**

(12) **EUROPÄISCHE PATENTSCHRIFT**

(45) Veröffentlichungstag der Patentschrift: **11.11.81**

(21) Anmeldenummer: **79102302.1**

(22) Anmeldetag: **06.07.79**

(51) Int. Cl.³: **C 07 D 487/18** //C08K5/34, (C07D487/18, 239/00, 251/00)

# (54) Verfahren zur Herstellung von 7-Nitro-1,3,5-triazaadamantan.

(30) Priorität: **19.07.78 DE 2831632**

(43) Veröffentlichungstag der Anmeldung: **23.01.80 Patentblatt 80/2**

(45) Bekanntmachung des Hinweises auf die Patenterteilung: **11.11.81 Patentblatt 81/45**

(84) Benannte Vertragsstaaten: **CH DE GB IT**

(56) Entgegenhaltungen:
**CH - A - 524 623**
**DE - A - 2 616 799**
**US - A - 3 624 253**
**US - A - 3 904 626**

**Chemical Abstracts, vol. 84 no. 14, 30. März 1976, Columbus, Ohio, USA M. Safar et al., "Nitrogen compounds of adamantane VIII Chromatographic separation of azaadamantanes and their derivatives in a gas liquid system" seite 620, Zeile 2 Abstrakt Nr. 98915 j**

(73) Patentinhaber: **HOECHST AKTIENGESELLSCHAFT**
**Postfach 80 03 20**
**D-6230 Frankfurt/Main 80 (DE)**

(72) Erfinder: **Wiezer, Hartmut, Dr.**
**Hans-Fischer-Strasse 6**
**D-8906 Gersthofen (DE)**

**EP 0 007 075 B1**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

Courier Press, Leamington Spa, England.

# Verfahren zur Herstellung von 7-Nitro-1,3,5-triazaadamantan

Die Erfindung betrifft ein verbessertes Verfahren zur Herstellung des durch die Formel (I) dargestellten 7-Nitro-1,3,5-triazaadamantans,

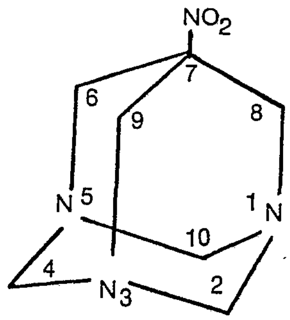

(I)

welches zum Stabilisieren von Kunststoffen geeignet ist und gleichzeitig Bedeutung als Ausgangsprodukt für die Synthese anderer Stabilisatoren besitzt.

Es ist bekannt, 7-Nitro-1,3,5-triazaadamantan durch Umsetzen von Paraformaldehyd mit Nitromethan und Ammonium-acetat in alkoholischem Medium herzustellen (US—A—3 301 854). Ein anderes Verfahren besteht in der Umsetzung von Tris-(Hydroxymethyl)-nitromethan mit wäßrigem Ammoniak und wäßriger Formaldehydlösung [E. B. Hodge, J. Chem. Soc. 37, 2 (1972) S. 320/321]. Schließlich ist auch noch ein Verfahren bekannt, nach welchem man Hexamethylentetramin mit Nitromethan und Ameisensäure in wäßrigem 80 % igem Ethanol bei höheren Temperaturen miteinander zur Reaktion bringt [M. Safar et al., Collection Czechoslov. Chem. Comm. 40 (1975) S. 2179—2182].

Bei dem erstgenannten Verfahren erhält man nicht, wie angegeben, Ausbeuten von 70 bis 77 %, sondern nur von 45 bis 55 %, was übereinstimmend von verschiedenen Autoren festgestellt wird [E. B. Hodge, loc. cit; A. T. Nielsen, J. Heterocyclic Chem. 12, 1 (1975) S. 161—164; A. R. Farminer et al., US NTIS AD — A Rep. 1975, Nr. 008097, S. 12]. Nach dem an 2. Stelle genannten Verfahren liegt die Ausbeute bei nur 28 %. Das letztgenannte Verfahren soll zwar mit einer Ausbeute von 65 % ablaufen, jedoch ist das erhaltene Produkt erheblich verunreinigt (vergl. hierzu Hydrierversuch c von Safar S. 2 180, mit demjenigen von Hodge, gemäß S. 321 linke Spalte 7. Absatz loc. cit. sowie Ergebnis der Nachstellung des Verfahrens von Safar). Dies ist besonders deshalb außerordentlich störend, weil dadurch eine Weiterverarbeitung zum 7-Amino-1,3,5,-triazaadamantan durch katalytische Hydrierung infolge Katalysatorvergiftung, nahezu unmöglich gemacht wird, wie die Hydrierergebnisse von Safar (z.B. Versuch a v. S. 2180) eindeutig zeigen. Aus der Veröffentlichung von Nielsen, (loc. cit., S. 161, Spalte 1, Zeilen 15—18) kann geschlossen werden, daß es sich bei dem von Safar erhaltenen Hydrierungsprodukt — wegen des drastisch abweichenden Schmelzpunktes — nur um Ausgangsmaterial handeln kann. Für die Herstellung von 7-Nitro-1,3,5-triazaadamantan im technischen Maßstab sind somit alle genannten Verfahren wenig geeignet.

Es wurde nun gefunden, daß man 7-Nitro-1,3,5-triazaadamantan überraschenderweise in guter Ausbeute und mit einem unerwartet hohen Reinheitsgrad erhalten kann, wenn man sich des letztgenannten Verfahrens in einer geänderten Form bedient.

Gegenstand der vorliegenden Erfindung ist daher ein Verfahren zur Herstellung von 7-Nitro-1,3,5-triazaadamantan durch Umsetzen von Hexamethylentetramin mit Nitromethan in Gegenwart eines sauren Katalysators in einem organischen Lösungsmittel bei erhöhter Temperatur, welches dadurch gekennzeichnet ist, daß das Lösungsmittel ein aliphatischer Alkohol mit 1 bis 4 C-Atomen mit einem Wassergehalt von nicht mehr als 4 Gew.-% oder eine wasserfreie aliphatische Monocarbonsäure mit 1 bis 3 C-Atomen ist, der saure Katalysator in einer dem Hexamethylentetramin mindestens äquimolaren Menge eingesetzt wird und man die Umsetzung bei 40 bis 180°C durchführt.

Es war nicht vorhersehbar, daß Hexamethylentetramin auch ohne den Zusatz von Wasser unter sauren Bedingungen mit Nitromethan zur Reaktion zu bringen sei, nachdem bekannt ist, daß bei den vom Hexamethylentetramin ausgehenden Verfahren letzteres sich zunächst mit Wasser und Säuren, z.B. Ameisensäure, zu Ammoniumformiat und Formaldehyd umsetzt nach der Gleichung:

$$C_6H_{12}N_4 + 6\ H_2O + 4\ H\text{-}COOH \rightleftharpoons 6\ CH_2O + 4\ H\text{—}COONH_4$$

und erst diese Reaktionsprodukte mit dem Nitromethan in der von Hodge, Nielsen und Farminer (loc. cit.) beschriebenen Weise zum Nitroazaadamantan reagieren. Die Umsetzung entsprechend der erfindungsgemäßen Arbeitsweise scheint somit nach einem völlig anderen Reaktionsmechanismus zu verlaufen.

Überraschend ist schließlich der hohe Reinheitsgrad des Verfahrensproduktes, welches aus den Reaktionslösungen in Gestalt gelblicher Kristalle abgeschieden wird, die man lediglich mit Wasser

nachwäscht und die ohne weitere Reinigungsmanipulation beispielsweise einer Hydrierung zur Aminoverbindung unterworfen werden können.

Hexamethylentetramin und Nitromethan werden bevorzugt in äquimolarem Verhältnis eingesetzt; ein Überschuß an der einen oder anderen Komponente bringt keine Vorteile.

Geeignete organische Lösungsmittel sind bevorzugt aliphatische Mono- oder Dialkohole mit 1 bis 4 bzw. 2 bis 4 C-Atomen, insbesondere Methanol und Ethanol, ferner Propanol, Isopropanol, n-Butanol, Ethylenglykol, Propylenglykol sowie aliphatische Monocarbonsäuren mit 1 bis 3 C-Atomen, bevorzugt Essigsäure, Wesentlich für das Gelingen des Verfahrens ist, daß die Alkohole nicht mehr als 4, vorzugsweise nicht mehr als 2 Gew.-% Wasser enthalten, da die Produktqualität mit steigendem Wassergehalt des Lösungsmittels merklich abnimmt, indem Nebenprodukte entstehen, die stärker gefärbt und kaum entfernbar sind. Die Lösungsmittelmenge liegt im allgemeinen beim 1-bis 10-fachen des Gewichts an eingesetztem Hexamethylentetramin.

Unter sauren Katalysatoren werden Protonensäuren, wie Chlorwasserstoff, organische Sulfon- oder Phosphonsäuren und insbesondere Carbonsäuren verstanden, im einzelnen z. B. Ameisensäure, Essigsäure, Propionsäure, oder p-Toluolsulfonsäure. Die Katalysatoren werden — bezogen auf eingesetztes Hexamethylentetramin- in ein- bis zehnfacher, vorzugsweise ein- bis fünf-facher und insbesondere ein- bis eineinhalbfacher molarer Menge verwendet. Wird als Lösungsmittel eine Carbonsäure eingesetzt, so erübrigt sich natürlich die Zugabe eines Katalysators; es ist dann jedoch darauf zu achten, daß die dem Hexamethylentetramin mindestens äquimolare Menge an diesem "Säure-Lösungsmittel" vorliegt.

Die Umsetzung wird bei Temperaturen von 40 bis 180, vorzugsweise 60 bis 140 und insbesondere 80 bis 130°C durchgeführt, wobei sich in Abhängigkeit von der Art des Lösungsmittels und der gewählten Temperatur ein Druck zwischen 1 und 20 bar einstellt. Die Reaktionszeiten hängen ebenfalls von der Reaktionstemperatur ab; sie liegen im allgemeinen zwischen einer und 25 Stunden.

Die folgenden Beispiele sollen die Erfindung näher erläutern. Die Löslichkeitstests wurden jeweils in heißem Wasser von 90—100°C durchgeführt.

Beispiel 1

140 g (1 Mol) Hexamethylentetramin, 61 g (1 Mol) Nitromethan und 200 g Eisessig werden 8 Stunden unter Rühren auf 95°C erhitzt. Nach ca. 30 Min. wird die Reaktion exotherm, wobei das Produkt auszukristallisieren beginnt. Nach Beendigung der Reaktionszeit wird abgenutscht und zweimal mit 150 ml Wasser gewaschen.

Ausbeute: 127 g = 69 % d. Th. gelbliche Kristalle.

Fp. 305—310°C (Z), 1 g restlos in 20 ml heißem Wasser löslich.

Beispiel 2

126 g Hexamethylentetramin, 65 g Nitromethan, 150 g Butanol und 60 g Eisessig werden 4 Stunden unter Rückfluß gekocht. Die ausgeschiedenen Kristalle werden sodann abgenutscht und mit zweimal 100 ml Wasser gewaschen.

Ausbeute: 114 g = 68 % d. Th., bezogen auf Hexamethylentetramin.

Fp. 285—295°C (Z), 1 g zu ca. 95 % in 20 ml heißem Wasser löslich.

Beispiel 3

140 g (1 Mol) Hexamethylentetramin, 65 g Nitromethan und 150 g Eisessig werden 4 Stunden auf 95°C gehalten. Es wird abgenutscht und mit zweimal 150 ml Wasser gewaschen.

Ausbeute: 129 g = 70 % d Th.

Fp. 305—310°C (Z), 1 g restlos in 20 ml heißem Wasser löslich.

Beispiel 4

70 g Hexamethylentetramin, 30,5 g Nitromethan und 25 g Ameisensäure werden in 250 ml 96 %igem Ethanol 20 Stunden am Rückfluß gekocht. Der ausgefallene Feststoff wird abgenutscht und mit Wasser gewaschen.

Ausbeute: 63,5 g = 69 % d. Th.

Fp. 295—305°C (Z), 1 g restlos in 20 ml heißem Wasser löslich.

Beispiel 5

70 g Hexamethylentetramin, 30,5 g Nitromethan und 60 g Eisessig werden in 250 ml Ethanol (96 %ig) 20 Stunden am Rückfluß gekocht. Die ausgefallenen Kristalle werden abgenutscht und mit Wasser gewaschen.

Ausbeute: 67,1 g = 72,8 % d. Th.

Fp. 295—305°C (Z), 1 g restlos in 20 ml heißem Wasser löslich.

Beispiel 6

70 g Hexamethylentetramin, 30,5 g Nitromethan und 30 g Eisessig werden in 250 ml absolutem Ethanol 20 Stunden am Rückfluß gekocht. Die ausgefallenen Kristalle werden abgenutscht und mit

Wasser gewaschen.
Ausbeute: 7,1 g = 78,4 % d. Th.
Fd. 295—305°C (Z), 1 g restlos in 20 ml heißem Wasser löslich.

Beispiel 7

560 g Hexamethylentetramin, 244 g Nitromethan und 400 g Eisessig werden in 1300 g Methanol (wasserfrei) in einem Druckgefäß 20 Stunden auf 80°C und anschließend 30 Minuten auf 140°C erhitzt. Der ausgefallene Feststoff wird nach dem Abkühlen abgenutscht, zweimal mit 500 ml Wasser gewaschen und getrocknet.
Ausbeute: 515 g = 70,0 % d. Th.
Fp. 295—305°C (Z), 1 g restlos in 20 ml heißem Wasser löslich.

Beispiel 8

70 g Hexamethylentetramin, 45 g Eisessig und 30,5 g Nitromethan werden in 250 ml Methanol 5 Stunden in einem Druckgefäß auf 100°C erhitzt. Der ausgefallene Feststoff wird abgenutscht, mit Wasser gewaschen und getrocknet.
Ausbeute: 67 g = 72,8 % d. Th.
Fp. 295—305°C (Z), 1 g restlos in 20 ml heißem Wasser löslich.

Beispiel 9

70 g Hexamethylentetramin, 30,5 g Nitromethan und 30 g Eisessig werden in 250 ml Methanol 3 Stunden in einem Druckgefäß unter Rühren auf 110°C erhitzt. Es werden 66 g = 71,7 % d. Th. der gewünschten Verbindung gewonnen.
Fp. 295—305°C (Z) 11 g restlos in 20 ml heißem Wasser löslich.

Beispiele 10 bis 12

Diese Beispiele zeigen, daß der Wassergehalt des verwendeten Lösungsmittels die Ausbeute an dem Verfahrensprodukt sowie dessen Qualität erheblich beeinflußt.

140 g Hexamethylentetramin (1 Mol), 61 g Nitromethan (1 Mol) und 90 g Eisessig (1,5 Mol) wurden in Gegenwart von 400 g Ethanol unterschiedlichen Wassergehaltes 22 Stunden unter Rückfluß gekocht. Es wurde heiß abgenutscht und getrocknet.

Die Ergebnisse sind in nachstehender Tabelle zusammengestellt. Die Löslichkeit in Wasser und das Verhalten beim Erhitzen stellen ein Maß für die Reinheit des Produktes dar.

| Bsp. Nr. | Gew.% $H_2O$ im Ethanol | Ausbeute (%) | Aussehen | Löslichkeit[1)] | Thermisches Verhalten[2)] |
|---|---|---|---|---|---|
| 10 | 4 | 142,6 g = 77,5 | gelblich | restlos | 300°C |
| 11 | 23 | 124,2 g = 67,5 | ocker | ca. 85 % | 280°C |
| 12 | 42,5 | 119,1 g = 64,7 | hell-braun | ca. 75 % | 270°C |

Beispiel 11 entspricht weitestgehend den Reaktions bedingungen die von M. Safar et al (loc. cit.) angegeben wurden. Die exakte Nachstellung ergab ein bräunlich gelbes Produkt, das bei 280°C schwarz wird und sich lediglich zu 82,5% in heißem Wasser löst (Bedingungen wie unter 1) angegeben.

1) 1 g Substanz soll in 20 ml heißem Wasser gelöst werden.
2) Temperatur, bei der das Produkt schwarz wird.

**Patentansprüche**

1. Verfahren zur Herstellung von 7-Nitro-1,3,5-triazaadamantan durch Umsetzen von Hexamethylentetramin mit Nitromethan in Gegenwart eines sauren Katalysators in einem organischen Lösungsmittel bei erhöhter Temperatur, dadurch gekennzeichnet, daß das Lösungsmittel ein aliphatischer Alkohol mit 1 bis 4 C-Atomen mit einem Wassergehalt von nicht mehr als 4 Gew.-% oder eine wasserfreie aliphatische Monocarbonsäure mit 1 bis 3 C-Atomen ist, der saure Katalysator in einer dem Hexamethylentetramin mindestens äquimolaren Menge eingesetzt wird und man die Umsetzung bei 40 bis 180°C durchführt.

2. Verfahren nach Anspruch 1, dadurch gekennzeichnet, daß man äquimolare Mengen Hexamethylentetramin und Nitromethan einsetzt.

3. Verfahren nach Anspruch 1, dadurch gekennzeichnet, daß das organische Lösungsmittel Methanol, Ethanol oder Eisessig ist.

4. Verfahren nach Anspruch 1, dadurch gekennzeichnet, daß der saure Katalysator eine wasserfreie aliphatische Monocarbonsäure mit 1 bis 3 C-Atomen ist, die gleichzeitig die Funktion des Lösungsmittels einnimmt.

## Claims

1. A process for the manufacture of 7-nitro-1,3,5-triaza-adamantane by reaction of hexamethylene tetramine with nitromethane in an organic solvent at elevated temperature in the presence of an acid catalyst, which comprises using as solvent an aliphatic alcohol having from 1 to 4 carbon atoms and a water content not exceeding 4 weight %, or an anhydrous aliphatic monocarboxylic acid having from 1 to 3 carbon atoms, employing the catalyst in an amount at least equimolar to that of the hexamethylene tetramine, and carrying out the reaction at 40 to 180°C.

2. The process as claimed in claim 1, which comprises using equimolar amounts of hexamethylene tetramine and nitromethane.

3. The process as claimed in claim 1, wherein the organic solvent is methanol, ethanol or glacial acetic acid.

4. The process as claimed in claim 1, wherein the acid catalyst is an anhydrous aliphatic monocarboxylic acid having from 1 to 3 carbon atoms which simultaneously acts as solvent.

## Revendications

1. Procédé de préparation de nitro-7 triaza-1,3,5 adamantane par réaction de l'hexaméthylènetétramine avec du nitrométhane en présence d'un catalyseur acide dans un solvant organique à une température élevée, procédé caractérisé en ce qu'on utilise comme solvant un alcool aliphatique en $C_1$—$C_4$ ayant une teneur en eau inférieure à 4 % en poids ou un acide monocarboxylique aliphatique anhydre en $C_1$—$C_3$, qu'on met en jeu le catalyseur acide en une quantité au moins équimolaire à l'hexaméthylène-tétramine et qu'on réalise la réaction à une température de 40 à 180°C.

2. Procédé suivant la revendication 1, caractérisé en ce qu'on met en jeu l'hexaméthylènetétramine et le nitrométhane en des quantités équimolaires.

3. Procédé suivant la revendication 1, caractérisé en ce qu'on utilise comme solvant organique le méthanol, l'éthanol ou l'acide acétique glacial.

4. Procédé suivant la revendication 1, caractérisé en ce qu'on utilise comme catalyseur acide un acide monocarboxylique aliphatique anhydre en $C_1$—$C_3$ jouant en même temps le rôle de solvant.